# EUROPEAN PATENT SPECIFICATION

(11) **EP 2 752 669 B1**
(45) Date of publication and mention of the grant of the patent: **27.04.2016**
(21) Application number: 14000001.9
(22) Date of filing: 02.01.2014
(51) Int. Cl.: G01N 35/10

(54) **Position correction device for tip portion of rod member**
Spitzepositionskorrekturvorrichtung
Appareil de correction de la position d'une extrémité d'une sonde

(30) Priority: 04.01.2013 JP 2013000158
(43) Date of publication of application: 09.07.2014
(73) Proprietor: Aoi Seiki Co., Ltd., Kumamoto-ken (JP)
(72) Inventor: Itoh, Teruaki, Kumamoto-shi, Kumamoto-ken (JP)
(74) Representative: Plöger, Jan Manfred

(56) References cited:
- EP-A1- 2 541 256
- WO-A1-92/15017
- WO-A1-2007/140531
- WO-A2-2012/158308
- US-A1- 2002 095 974

## Description

### 1. Field of the Invention

The present invention relates to, for example, an examination pretreatment apparatus that moves a sample in one container to another container.

### 2. Description of the Related Art

In the conventional art, when an examination such as a biochemical analysis is carried out on the mucous membrane in the oral cavity, first, the mucous membrane is sampled with a cotton swab. Then, the cotton swab with the mucous membrane is stored in a container. Before the examination such as a biochemical analysis is carried out, a pretreatment is performed in which the mucous membrane attached to the cotton swab is transferred into a container that contains saline. After the container that contains the saline with the mucous membrane is carried to an apparatus for each examination, the examination is performed.

The cotton swab is shaped like a long rod and may thus be bent. Since the cotton swab may be bent, the position of a tip portion to which the mucous membrane is attached varies among cotton swabs. Thus, to insert the tip portion with the mucous membrane attached thereto into the container that contains the saline, it is necessary to visually check whether or not the tip portion of the cotton swab is in the correct position, while manually inserting the tip portion into the container. This reduces work efficiency.

On the other hand, an examination pretreatment apparatus has been proposed which automatically carries out pretreatments such as various blood tests. An examination pretreatment apparatus of this kind is disclosed in Jpn. Pat. Appln. KOKAI Publication No. 2012-52872. The automatic treatment using the apparatus improves the work efficiency.
WO 92/15017 A1 describes a method of calibration pipette position in an automated assay instrument adapted for use with disposable pipette tips. An electro-optic detector is used to detect the position of the pipette, and a microprocessor is used for controlling the position of the pipette.
US 2002/0095974 A1 discloses a probe drive systems of a precision liquid handler that sequentially inserts probe tips into a locator well on a locator bed to determine the relative positions of the different probe tips. The position of each probe tip is determined by driving the probe tip into contact with points of the side wall of the locator well. Misalignments of the probe tips can be corrected by a controller and a drive system.
WO 2007/140531 A1 discloses a swab device adapted to be securely gripped by automated or semi-automated transfer means for the transfer of the swab device between stations. A holder is designed to enable the gripping means to register more precisely with the body of the swab device and transfer the swab device to a predefined location in a processing station without misalignment of the sample due to slippage etc. in the gripping means of the transfer device.
EP 2 541 256 A1 discloses an apparatus for correcting the position of a nozzle tip end by using an inclination correction mechanism. The position correction occurs in a passive way using resilient members.
WO 2012/158308 A2 discloses a sensor to detect the position of a pipette with respect to well locations. This is achieved by using an array of infrared photodiodes that produce beams of light sent to walls photoreceptors.

### BRIEF SUMMARY OF THE INVENTION

It has been desired to efficiently carry out a
pretreatment in which a sample attached to the tip portion of a long rod member such as a cotton swab in one container is transferred into another container.

An object of the present invention is to provide an examination pretreatment apparatus that can efficiently carry out the pretreatment in which the sample attached to the tip portion of the rod member in one container is transferred into another container.

An examination pretreatment apparatus according to the invention comprising: moving means for placing a tip portion of a rod member housed in a first container in a second container different from the first container through an opening in the second container; position detection means for detecting a position of the tip portion; position correction means for correcting the position of the tip portion by tilting the moving means; and control means configured to control the position correction means so that the tip portion is moved into the second container, based on a result of detection by the position detection means, and to control the position correction means so as to swing the tip portion inside the second container after the tip portion is housed in the second container.

The present invention allows efficient execution of the pretreatment in which the sample attached to the tip portion of the member in one container is transferred into another container.

### BRIEF DESCRIPTION OF THE SEVERAL VIEWS OF THE DRAWINGS

The accompanying drawings, which are incorporated in and constitute a part of the specification, illustrate embodiments of the invention, and together with the general description given above and the detailed description of the embodiments given below, serve to explain the principles of the invention.
FIG. 1 is a schematic diagram showing a part of an examination system in which an examination pretreatment apparatus according to an embodiment of the present invention is incorporated;
FIG. 2 is a cross-sectional view of the examination system shown along line F2-F2 in FIG. 1;
FIG. 3 is a perspective view of a chuck section of the examination pretreatment apparatus as seen from below the apparatus;
FIG. 4 is a schematic diagram showing an operation in which a chuck device of the examination pretreatment apparatus transfers the mucous membrane attached to a cotton swab of a cap member into a first container;
FIG. 5 is a plan view showing that a tip portion of the cotton swab overlaps the inside of an opening in the first container in an up-down direction;
FIG. 6 is a perspective view showing how the tip portion is housed in the first container;
FIG. 7 is a schematic diagram showing how the examination pretreatment apparatus operates when the cotton swab is bent;
FIG. 8 is a plan view showing that, when the cotton swab is bent, the tip portion has moved until the position of the tip portion is detected by a position detection device;
FIG. 9 is an enlarged front view of a position correction apparatus of the examination pretreatment apparatus; and
FIG. 10 is an enlarged front view of the position correction apparatus of the examination pretreatment apparatus.

### DETAILED DESCRIPTION OF THE INVENTION

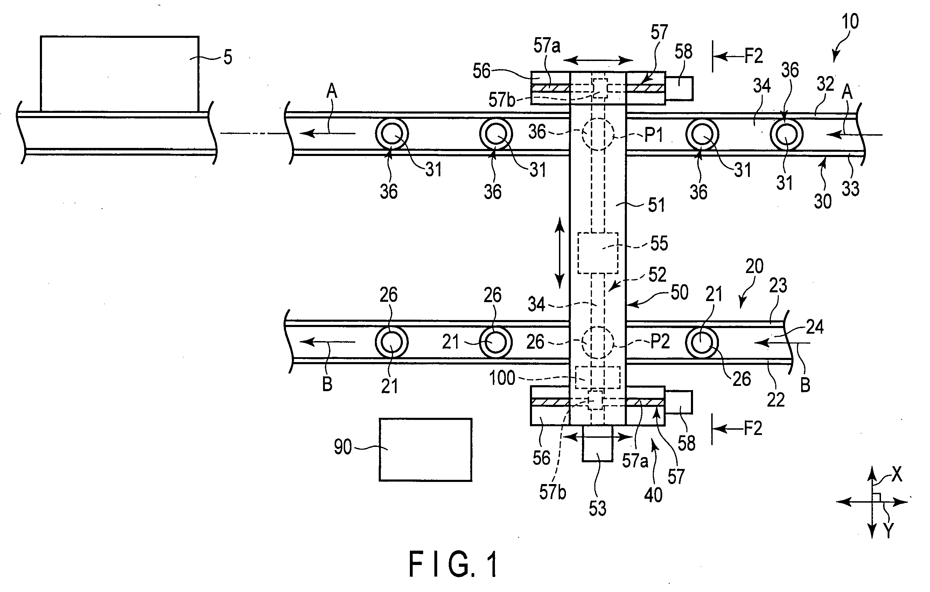

An examination pretreatment apparatus according to an embodiment of the present invention will be described with reference to FIG. 1 to FIG. 10. FIG. 1 is a schematic diagram showing a part of an examination system 10 in which an examination pretreatment apparatus 40 is incorporated. The examination system 10 examines, by way of example, the mucous membrane in the human oral cavity according to the present embodiment.

As shown in FIG. 1, the examination system 10 comprises a plurality of examination apparatuses, a first conveying path 20, a second conveying path 30 along which the mucous membrane, an example of a sample, is conveyed to each of the examination apparatuses, and an examination pretreatment apparatus 40. FIG. 1 shows the vicinity of the examination pretreatment apparatus 40 in the examination system 10. FIG. 1 shows a part of the first conveying path 20 which is disposed in the vicinity of the examination pretreatment apparatus 40, with the remaining part of the first conveying path 20 omitted.

FIG. 1 shows a part of the second conveying path 30 located in the vicinity of the examination pretreatment apparatus 40 and a part of the second conveying path 30 located in the vicinity of an examination apparatus 5 that is a part of the plurality of examination apparatuses. The remaining part of the second conveying path 30 is omitted.
According to the present embodiment, the examination system 10 comprises, by way of example, the plurality of examination apparatuses, but only the examination apparatus 5 is shown. The examination apparatuses carry out different examinations on the mucous membrane, which is a sample.

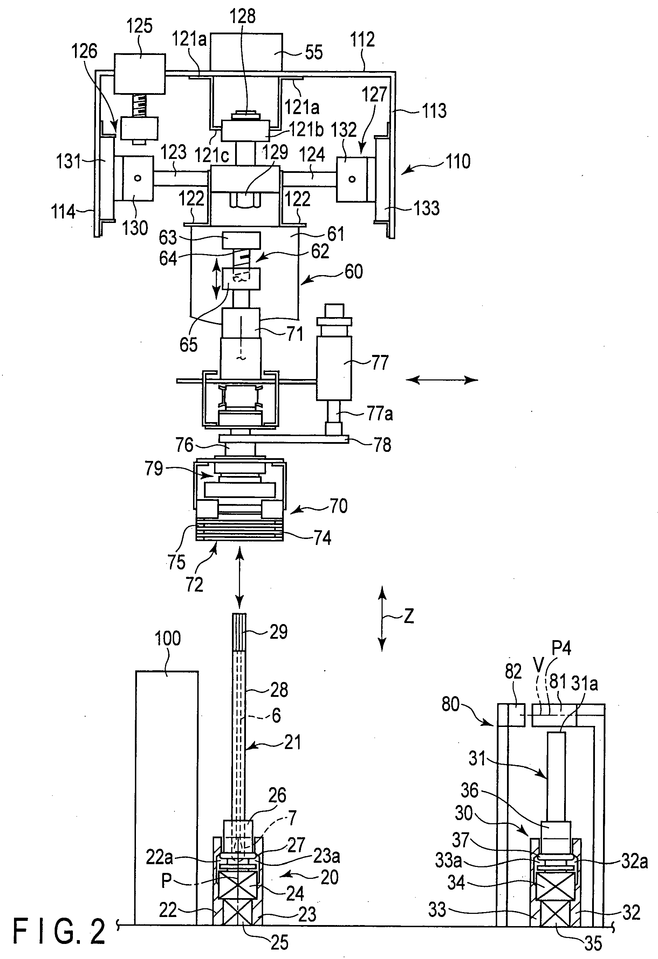

First, the second conveying path 30 will be described. The second conveying path 30 conveys a second holder that contains a sample to each examination apparatus. FIG. 2 is a cross-sectional view of the examination system 10 shown along line F2-F2 shown in FIG. 1. FIG. 2 shows the examination pretreatment apparatus 40 as viewed from front and the first and second conveying paths 20 and 30 as severed.

As shown in FIG. 2, the second conveying path 30 comprises a pair of guide rails 32 and 33, a second conveying belt 34, a second conveying belt driving section 35, and a plurality of second holders 36. As shown in FIGS. 1 and 2, the paired guide rails 32 and 33 are disposed opposite each other. The guide rails 32 and 33 are shaped like vertical walls extending in an up-down direction Z. The guide rails 32 and 33 extend so as to connect the examination apparatuses together in order to convey the second holder 36 described below to each of the examination apparatuses.

The second conveying belt 34 is provided between the guide rails 32 and 33 and can move along the guide rails 32 and 33. Specifically, the second conveying belt 34 is disposed between the guide rails 32 and 33 so that an upper surface of the second conveying belt 34 extends in a direction perpendicular to the up-down direction Z. The second conveying belt driving section 35 comprises, for example, a plurality of rollers and has a function to feed the second conveying belt 34.

Each of the second holders 36 is shaped like a cylinder with an open upper end. The second holder 36 internally houses a second container 31 so that the second container 31 is removable from the second holder 36. The second holder 36, connected to the guide rails 32 and 33, lies on the second conveying belt 34. Thus, when the second conveying belt 34 is fed along the guide rails 32 and 33, the second holder 36 is fed along the guide rails 32 and 33 along with the second conveying belt 34. In FIG. 1, a direction A in which the second conveying belt 34 and the second holder 36 are fed is shown by an arrow.

A structure in which the second holder 36 is connected to the guide rails 32 and 33 will be specifically described. As shown in FIG. 2, opposite sidewall portions of the guide rails 32 and 33 comprise recess portions 32a and 33a, respectively, formed in the sidewall portions and recessed outward. The recess portions 32a and 33a are continuously formed in a direction in which the guide rails 32 and 33 extend. Upper ends of the recess portions 32a and 33a function as stopper portions.

An engagement portion 37 projecting outward in a circumferential direction is formed on a circumferential wall portion of each of the second holders 36. When the second holder 36 is placed on the second conveying belt 34, the engagement portion 37 is housed in the recess portions 32a and 33a of the guide rails 32 and 33.

When an attempt is made to remove the second holder 36 from between the guide rails 32 and 33 by moving the second holder 36 upward, the upper ends of the recess portions 32a and 33a, functioning as stopper portions, engage with the engagement portion 37. This prevents the second holder 36 from being removed from between the guide rails 32 and 33. Thus, the second holder 36 is connected to the guide rails 32 and 33. The second holders 36 are disposed at a predetermined distance from each other.

The second container 31 is shaped like a cylinder with an open upper end. According to the present embodiment, the second container 31 contains, by way of example, saline. Furthermore, the examination pretreatment apparatus 40 transfers the mucous membrane, an example of a sample, from a first container 21 described below into the second container 31. The second holder 36 that houses the second container 31 to which the mucous membrane has been supplied by the examination pretreatment apparatus 40 is carried to the examination apparatus by the second conveying path 30.

The first conveying path 20 conveys the first container 21 that contains the mucous membrane, to the examination pretreatment apparatus 40. As shown in FIG. 2, the first conveying path 30 comprises a pair of guide rails 22 and 23, a first conveying path 24, a first conveying belt driving section 25, and a plurality of first holders 26. As shown in FIGS. 1 and 2, the paired guide rails 22 and 23 are disposed opposite each other. The guide rails 22 and 23 are shaped like vertical walls extending in the up-down direction Z.

The first conveying belt 24 is provided between the guide rails 22 and 23 and can move along the guide rails 22 and 23. Specifically, the first conveying belt 24 is disposed between the guide rails 22 and 23 so that an upper surface of the second conveying belt 24 extends in a direction perpendicular to the up-down direction Z. The first conveying belt driving section 25 comprises, for example, a plurality of rollers and has a function to feed the first conveying belt 24.

Each of the second holders 26 is shaped like a cylinder with an open upper end. The first holder 26 internally houses a first container 31 so that the first container 31 is removable from the first holder 26. The first holder 26, connected to the guide rails 22 and 23, lies on the first conveying belt 24. Thus, when the first conveying belt 24 is fed along the guide rails 22 and 23, the first holder 26 is fed along the guide rails 22 and 23 along with the first conveying belt 24. In FIG. 1, a direction B in which the first conveying belt 24 and the first holder 26 are fed is shown by an arrow.

The plurality of first holders 26, disposed at a predetermined distance from each other, are connected to the guide rails 22 and 23 so as to be prevented from rotating. The term "rotation" as used herein refers to a rotation around the centerline P of the first holder 26.

A structure in which the first holder 26 is connected to the guide rails 22 and 23 will be specifically described. As shown in FIG. 2, opposite sidewall portions of the guide rails 22 and 23 comprise recess portions 22a and 23a, respectively, formed in the sidewall portions and recessed outward. The recess portions 22a and 23a are continuously formed in a direction in which the guide rails 22 and 23 extend. Upper ends of the recess portions 22a and 23a function as stopper portions.

An engagement portion 27 projecting outward in the circumferential direction is formed on a circumferential wall portion of each of the first holders 26. When the first holder 26 is placed on the first conveying belt 24, the engagement portion 27 is housed in the recess portions 22a and 23a of the guide rails 22 and 23. Moreover, when an attempt is made to remove the first holder 26 from between the guide rails 22 and 23 by moving the second holder 26 upward, the upper ends of the recess portions 22a and 23a, functioning as stopper portions, engage with the engagement portion 27. This prevents the first holder 26 from being removed from the guide rails 22 and 23.

The first holder 21 comprises a container main body 28 shaped like a cylinder with an open upper end and a cap member 29. An external thread portion is formed at an edge of an opening in the container main body 28. The cap member 29 comprises an internal thread portion formed thereon to engage with the external thread portion in a threaded manner. The cap member 29 engaged with the container main body 28 in a threaded manner closes the opening in the container main body 28.

A cotton swab 6 is fixed to the cap member 29. The cotton swab 6 projects from the cap member 29. While the cap member 29 is engaged with the container main body 28 in a threaded manner, the cotton swab 6 is housed inside the container main body 28. The cotton swab 6 is shaped to be elongate in one direction. Thus, the container main body 28 is shaped to be elongate in one direction in order to house the cotton swab 6 inside the container main body 28 with the cap member 29 completely engaged with the container main body 28 in a threaded manner. The mucous membrane, an example of a sample, is attached to a tip portion of the cotton swab 6.

The first conveying path 20 comprises a fixation mechanism to which the container main body 28 is selectively fixed. Fixing the fixation mechanism to the container main body 28 allows the position and orientation of the container main body 28 to be fixed with respect to the first conveying path 20. Even when the container main body 28 is fixed by the fixation mechanism, the cap member 29 is not fixed. Thus, the cap member 29 can be rotated relative to the fixed container main body 28.

The first holder 26 holds the first container 21 so as to orient the first container 21 such that the cotton swab 6 extends parallel to the up-down direction Z. The plurality of first holders 26 are connected to the guide rails 22 and 23 so as to lie at a predetermined distance from each other.

The examination pretreatment apparatus 40 has a function to transfer the mucous membrane contained in the first container 21 into the second container 31. This will be specifically described. The mucous membrane is sampled with the cotton swab 6 outside the examination system 10. Thus, the first container 21, in which the externally sampled mucous membrane is contained, needs to be set to a state suitable for examinations carried out in the respective examination apparatuses. According to the present embodiment, the state suitable for the examination apparatuses is a state in which the mucous membrane is contained in the container along with saline. Thus, as a pretreatment, the examination pretreatment apparatus 40 transfers the mucous membrane attached to the cotton swab 6 of the first container 21 into the second container 31.

As shown in FIG. 1 and FIG. 2, the examination pretreatment apparatus 40 comprises a first moving device 50, a second moving device 60, a chuck device 70 removably attached to the cap member 29 of the first container 21, a position detection device 80, a position correction device 110, and a control device 90.

The first moving device 50 moves the chuck device 70 in a first direction X and a second direction Y which intersect each other. As shown in FIG. 1, the first direction X is a direction from the first conveying path 20 toward the second conveying path 30 and a direction from the second conveying path 30 toward the first conveying path 20. The second direction Y is perpendicular to the first direction X and also to the up-down direction Z. The up-down direction Z is, by way of example, a direction in which gravity acts and a direction opposite to the direction in which gravity acts.

The first moving device 50 comprises a first base portion 51 extending from the second conveying path 30 to the first conveying path 20 along the first direction X, a first ball screw device 52, and a first driving motor 53. The first ball screw device 52 comprises a first thread portion 54 and a first nut portion 55.

The first thread portion 54 is rotatably supported on the first base portion 51 and extends along the first direction X. The first nut portion 55 is fixed to the chuck device 70, described below, via the second moving device 60, described below, and is engaged with the first thread portion 54 in a threaded manner. The first driving motor 53 rotates the first thread portion 54 around the axis of the first thread portion 54. Rotation of the first thread portion 54 moves the first nut portion 55 over the first thread portion 54. This enables the chuck device 70 fixed to the first nut portion 55 to move from the second conveying path 30 side to the first conveying path 20 side and from the first conveying path 20 side to the second conveying path 30 side.

Furthermore, the first moving device 50 comprises a pair of second base portions 56, a pair of second ball screw devices 57, and a pair of second driving motors 58. One of the second base portions 56 is disposed opposite the second conveying path 30 across the first conveying path 20. The other second base portion 56 is disposed opposite the first conveying path 20 across the second conveying path 30. Both second base portions 56 extend in the second direction Y.

One of the second ball screw devices 57 is provided in one of the second base portions 56. The other second ball screw device 57 is provided in the.other second base portion 56. Both second ball screw devices 57 are the same.

The second ball screw device 57 comprises a second thread portion 57a and a second nut portion 57b. The second thread portion 57a is rotatably provided in the second base portion 56, and extends along the base portion 56. That is, the second thread portion 57a extends in the second direction Y. The second nut portion 57b is rotatably engaged with the second thread portion 57a in a threaded manner. Furthermore, both second nut portions 57b are fixed to the first base portion 51.

One of the second driving motors 58 rotates one of the second thread portions 57a. The other second driving motor 58 rotates the other second thread portion 57a. Both second driving motors 58 operate in the same manner by the control of the control device 90, described below. The second nut portion 57b, driven by the second driving motor 58, moves over the second thread portion 57a.

This allows the first base portion 51 fixed to the second nut portion 57b to move in the second direction Y. Since both the second driving motors 58 operate in the same manner as described above, the first base portion 51 moves in the second direction Y while remaining oriented to extend parallel to the first direction X. The chuck device 70 can thus be moved in the first and second directions X and Y by the first moving device 50.

The second moving device 60 moves the chuck device 70 in the up-down direction Z. In other words, the second moving device 60 moves the chuck device 70 in directions orthogonal to the first and second directions X and Y. As shown in FIG. 2, the second moving device 60 comprises a third base portion 61, a third ball screw device 62, and a third driving motor 63.

The second moving device 60 is fixed to the first moving device 50 via the position correction device 110, described below. Specifically, the third base portion 61 is fixed to the first nut portion 55 of the first moving device 50 via the position correction device 110, described below. The third base portion 61 moves together with the first nut portion 55. The third base portion 61 extends in the up-down direction Z. For easy understanding of the chuck device 70, FIG. 2 shows an upper end of the third base portion 61, with the remaining part thereof omitted.

The third ball screw device 62 comprises a third thread portion 64 and a third nut portion 65. The third thread portion 64 is rotatably supported by the third base portion 61, and extends in the up-down direction Z. FIG. 2 shows an upper end of the third thread portion 64, with the remaining part thereof omitted.

The third nut portion 65 is engaged with the third thread portion 64 in a threaded manner. The third driving motor 63 rotates the third thread portion 64 around the axis of the third thread portion 64. The third nut portion 65, driven by the third driving motor 63, moves over the third thread portion 64. As described above, the chuck device 70 is fixed to the third nut portion 65. This allows the chuck device 70 to move along the up-down direction Z. The chuck device 70 can thus be moved in the up-down direction Z and the first and second directions X and Y by the first and second moving device 50 and 60.

The present embodiment uses the first and second moving devices 50 and 60 as an example of means for moving the chuck device 70 in the up-down direction Z and the first and second directions X and Y. In another example, the chuck device 70 can be moved in the up-down direction Z and the first and second directions X and Y by a robot arm.

The chuck device 70 comprises a fixed portion 71 fixed to the third nut portion 65, a chuck section 72 that chucks the cap member 29 of the first container 21 so that the cap member 29 is removable from the chuck section 72, a fourth driving motor 77 that is an example of a rotation device rotating the chuck section 72 around the up-down direction Z, a transmission belt 78 that is an example of a transmission mechanism, a rotating section 76, and a conversion mechanism 79.

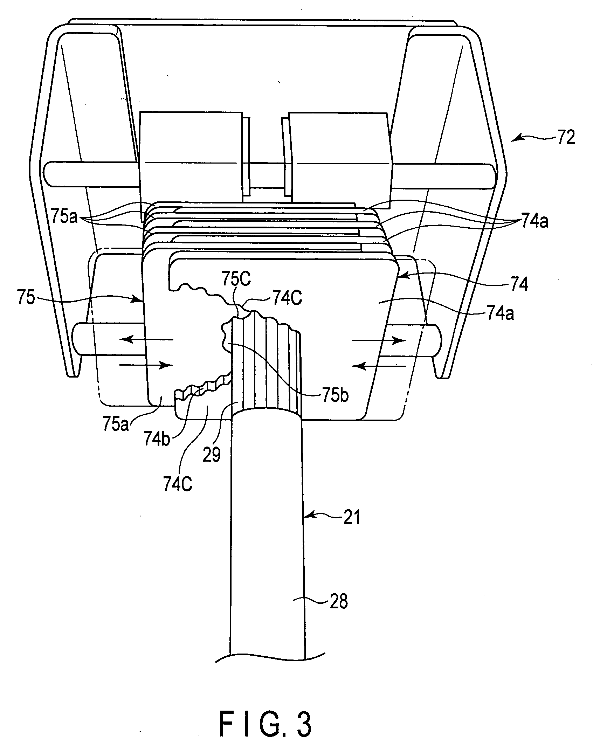

FIG. 3 is a perspective view showing the chuck section 72 as seen from below the apparatus. FIG. 3 shows how the chuck section 72 grips the cap member 29 of the first container 21. As shown in FIG. 2 and FIG. 3, the chuck section 72 comprises a first sandwich section 74, and a second sandwich section 75.

The chuck section 72 grips the cap member 29 so that the cap member 29 is sandwiched between the first and second sandwich sections 74 and 75. In this state, the first sandwich section 74 has moved toward the second sandwich section 75, and the second sandwich section 75 has moved toward the first sandwich section 74. The first sandwich section 74 comprises a plurality of first plate potions 74a spaced from one another in the up-down direction Z. The present embodiment includes, by way of example, four first plate portions 74a fixed together. The first plate portion 74a is rectangular in plan.

A first housing groove 74b is formed in the first plate portion 74a. The first housing groove 74b is open toward the second sandwich section 75 and penetrates the first plate portion 74a in a thickness direction thereof. The first housing groove 74b is shaped to decrease in size with increasing distance from the second sandwich section 75. The first housing groove 74b is generally triangular in plan.

The second sandwich section 75 comprises a plurality of second plate portions 75a. The second plate portion 75a is rectangular in plan. Each of the second plate portions 75a is disposed between the adjacent first plate portions 74a. The present embodiment includes, by way of example, four second plate portions 75a.

A second housing groove 75b is formed in the second plate portion 75a. The second housing groove 75b has a shape opposite to the first housing groove 74b of the first plate portion 74a, and is open toward the first sandwich section 74. The second housing groove 75b penetrates the second plate portion 75a in a thickness direction thereof. Since the first and second plate portions 74a and 75a are arranged in the up-down direction Z, the first and second housing grooves 74a and 75b are in communication with one another in the up-down direction Z.

The cap member 29 of the first container 21 is housed in the first and second housing grooves 74b and 75b in the first and second plate portions 74a and 75a. When the first sandwich sections 74 are displaced to move closer to the second sandwich sections 75 and vice versa, that is, when the first and second sandwich sections 74 and 75 move to the respective positions shown in FIG. 3, the cap member 29 is sandwiched between edges 74c and 75c of the first and second housing grooves 74b and 75b.

Small recesses and protrusions are alternately formed on the edges 74c and 75c of the first and second housing grooves 74b and 75b. Furthermore, recesses and protrusions are alternately formed on a circumferential surface portion of the cap member 29 along a circumferential direction of the cap member 29. Since the cap member 29 is sandwiched between the edges 74c and 75c of the first and second housing grooves 74b and 75b of the first and second plate portions 74a and 75a, the recesses and protrusions of the edges 74c and 75c mesh with the protrusions and recesses of the cap member 29. This allows the cap member 29 to be firmly fixed between the first sandwich sections 74 and the second sandwich sections 75. A structure for moving the first and second sandwich sections 74 and 75 forward and backward will be specifically described below.

As shown in FIG. 2, an output shaft 77a of the fourth driving motor 77 is connected to the rotating section 76 via the transmission belt 78. Specifically, the transmission belt 78 is passed around the output shaft 77a and the rotating section 76. Rotation of the output shaft 77a of the fourth driving motor 77 is transmitted to the rotating section 76 via the transmission belt 78. The rotating section 76 is rotatably connected to the fixed portion 71

The conversion mechanism 79 is connected to the rotating section 76 and to the chuck section 72. The conversion mechanism 79 converts rotation of the rotating section 76 into an opening and closing operation of the first and second sandwich sections 74 and 75 or a rotating operation of the chuck section 72. While the rotating section 76 is in an initial position, the conversion mechanism 79 places the first and second sandwich sections 74 and 75 in an open position. The open position is a position shown by an alternate long and two short dashes line in FIG. 3 and in which the second sandwich section 75 is located furthest from the first sandwich section 74, whereas the first sandwich section 74 is located furthest from the second sandwich section 75.

When the rotating section 76 makes a predetermined rotation from the initial position, that is, when rotation of the output shaft 77a is transmitted via the transmission belt 78 to the rotating section 76, which thus makes the predetermined rotation, the conversion mechanism 79 moves the first and second sandwich sections 74 and 75 to a closed position without rotating the chuck section 72. The closed position is a position in which the first sandwich section 74 is located closest to the second sandwich section 75, whereas the second sandwich section 75 is located closest to the first sandwich section 74. When the first and second sandwich sections 74 and 75 move to the closed position, the cap member 29 is fixedly sandwiched between the edges 74c and 75c of the first and second housing grooves 74b and 75b.

Furthermore, when the first and second sandwich sections 74 and 75 are stopped after starting to move toward the closed position, the conversion mechanism 79 converts the rotation of the rotating section 76 into rotation of the chuck section 72 around the up-down direction Z. When the first and second sandwich sections 74 and 75 are stopped after starting to move toward the closed position, the first and second sandwich sections 74 and 75 may have moved to the closed position with nothing sandwiched between the first and second sandwich sections 74 and 75 or the cap member 29 or the like is placed and sandwiched between the first and second sandwich sections 74 and 75. At this time, the direction of the rotation is the direction in which the cap member 29 threadably engaged with the container main body 28 of the first container 21 in a threaded manner is removed from the container main body 28.

Furthermore, when the rotating section 76 rotates in a direction opposite to the direction described above, the conversion mechanism 79 converts the rotation of the rotating section 76 such that the chuck section 72 rotates the cap member 29 in a direction in which the cap member 29 engages with the container main body 28 of the first container 21 in a threaded manner and then moves the first and second sandwich section 74 and 75 toward the position where the first and second sandwich sections 74 and 75 are opened.

The position detection device 80 is disposed on the first conveying path 20. The position detection device 80 detects the position of a tip portion 7 of the cotton swab 6 fixed to the cap member 29.

Specifically, the position detection device 80 comprises a first direction position detection section 81 that detects the position of the tip portion 7 of the cotton swab 6 along the first direction X and a second direction position detection section 82 that detects the position of the tip portion 7 along the second direction Y. The first direction position detection section 81 is shaped to extend in the first direction X. The second direction position detection section 82 is shaped to extend in the second direction Y.

Furthermore, the first direction position detection section 81 and the second direction position detection section 82 have a predetermined length along the up-down direction Z. The length, along the up-down direction Z, of the first and second direction position detection sections 81 and 82 will be specifically described below.

The position correction device 110 corrects the position of the tip portion 7 of the cotton swab 6 by tilting the second moving device 60. As shown in FIG. 2, the position correction device 110 is connected to the first nut portion 55 and to the second moving device 60. Thus, the second moving device 60 is connected to the first moving device 50 via the position correction device 110.

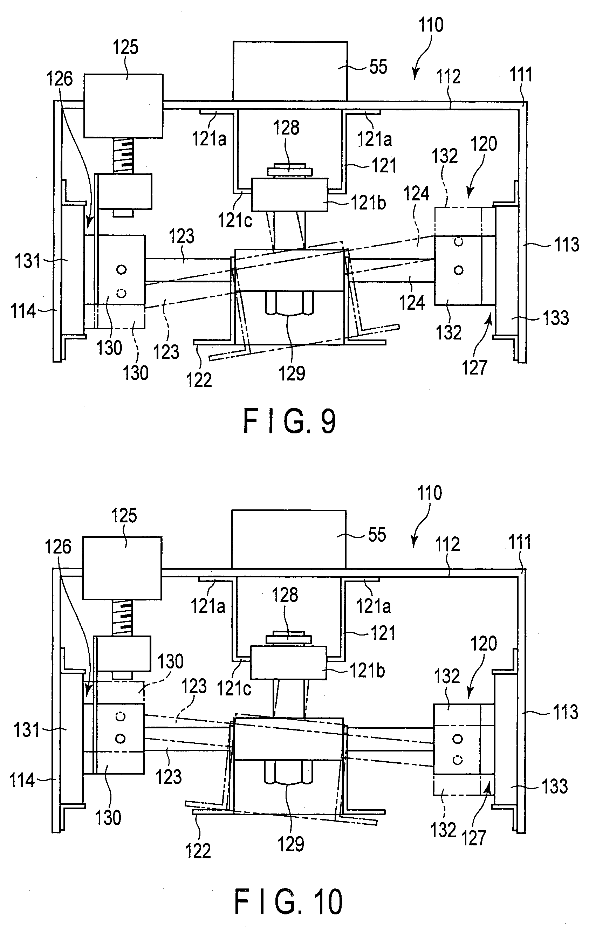

FIG. 9 is an enlarged front view of the position correction device 110. As shown in FIG. 9, the position correction device 110 comprises a frame member 111 fixed to the first nut portion 55, and a tilt mechanism 120 fixed to the frame member 111. The frame member 111 is shaped like, by way of example, a recess that is open downward.

The tilt mechanism 120 comprises a bracket 121 fixed to a bottom wall portion 112 of the frame member 111, a fixed portion 122 to which the second moving device 60 is fixed, a first arm portion 123 and a second arm portion 124 provided on the fixed portion 122 so as to extend outward, a fourth ball screw device 125 that tilts the fixed portion 122 relative to the frame member 111, a first support mechanism 126 that supports the first arm potion 123 so that the first arm position 123 is movable in the up-down direction Z, and a second support mechanism 127 that supports the second arm potion 124 so that the second arm position 124 is movable in the up-down direction Z.

The bracket 121 is shaped like, for example, a recess that is open upward, and comprises a flange portion 121a stretching out from an upper end of the bracket 121 to the surrounding space. The flange 121a is fixed to the bottom wall portion 112 of the frame member 111. Thus, the bracket 121 projects downward from the bottom wall portion 112.

The fixed portion 122 is supported to the bracket 21 so as to be able to tilt in the up-down direction. According to the present embodiment, the fixed portion 122 is supported by the bracket 121 via a cylindrical member 121b formed of rubber and which is elastically deformable. According to the present embodiment, the fixed portion 122 is fixed to the bracket 121 by a bolt 129.

The cylindrical portion 121b is fixed to a bottom wall portion 121c corresponding to a lower end of the bracket 121. The bolt 129 penetrates the cylindrical member 121b. A tip portion of the bolt 129 penetrating the cylindrical portion 121b is engaged with a nut 128 in a threaded manner. The nut 128 engages with the tip portion of the bolt 129 in a threaded manner to prevent the bolt 129 from slipping out. Thus, the fixed portion 122 is supported by the frame member 111 via the bracket 121. The third base portion 61 of the second moving device 60 is fixed to the fixed portion 122.

The first arm portion 123 projects from the fixed portion 122 in a direction parallel to the first direction X and extends parallel to the first direction X. The second arm portion 124 projects from the fixed portion in a direction parallel to the first direction X and extends parallel to the first direction X. The direction in which first arm portion extends is opposite to the direction in which the second arm portion extends.

The first support mechanism 126 is provided on a side wall portion 114 of the frame member 111 to support the tip portion of the first arm portion 123 on the side wall portion 114 so that the tip portion is movable in the up-down direction Z. The side wall portion 114 is a wall portion located opposite the tip portion of the first arm portion 123. The first support mechanism 126 comprises a first tip support section 130 rotationally movably connected to the tip portion of the first arm portion 123, and a first rail 131 that supports the first tip support section 130 so that the first tip support section 130 is movable in the up-down direction Z. The first rail 131 is fixed to the side wall portion 114.

The second support mechanism 127 is provided on a side wall portion 113 of the frame member 111 to support the tip portion of the second arm portion 124 on the side wall portion 113 so that the tip portion is movable in the up-down direction Z. The side wall portion 113 is a wall portion located opposite the tip portion of the second arm portion 124. The second support mechanism 127 comprises a second tip support section 132 rotationally movably connected to the tip portion of the second arm portion 124, and a second rail 133 that supports the second tip support section 132 so that the second tip support section 132 is movable in the up-down direction Z. The second rail 133 is fixed to the side wall portion 113.

The fourth ball screw device 125 is connected to the first tip support section 130 to enable the first tip support section 130 to be moved in the up-down direction Z.

Now, operation of the position correction device 110 will be described. When the fourth ball screw device 125 moves the tip portion of the first arm portion 123 downward, the first arm portion 123 is tilted relative to the initial position shown by a solid line, as shown by an alternate long and two short dashes line in FIG. 9. Since the fixed portion 122 is fixed to the frame member 111 via the cylindrical portion 121b, which is elastically deformable, the cylindrical portion 121b is deformed as the orientation of the first arm portion 123 shown by the alternate long and two short dashes line changes. Thus, the orientation of the fixed portion 122 is also made oblique.

When the'orientation of the fixed portion 122 is made oblique, the second arm portion 124, fixed to the fixed portion 122, is also tilted. The first and second arm portions 123 and 124 extend so as to be linearly connected together with the fixed portion 122 sandwiched between the first and second arm portions 123 and 124. Thus, the second arm portion 124 is tilted such that the tip portion moves upward relative to the initial position shown by a solid line.

Since the tip portions of the first and second arm portions 123 and 124 are rotationally movably connected to the tip support sections 130 and 132 of the first and second support mechanisms 126 and 127, respectively, even when the orientations of the first and second arm portions 123 and 124 are made oblique, the first and second arm portions 123 and 124 rotate relative to the first and second tip support sections 130 and 132, respectively, in conjunction with the change in the orientations of the first and second arm portions 123 and 124. This prevents, for example, a possible twist between the first and second arm portions 123 and 124 and the first and second support mechanisms 126 and 127.

FIG. 10 shows that the fixed portion 122 has been tilted in a direction opposite to the direction shown in FIG. 9. In a state shown in FIG. 10, the fourth ball screw device 125 moves the tip portion of the first arm portion 123 upward relative to an initial position shown by a solid line, thus tilting the first and second arm portions 123 and 124 in a direction opposite to the direction shown in FIG. 9. This causes the fixed portion 122 to be tilted in the direction opposite to the direction shown in FIG. 9.

The orientation of the fixed portion 122 can be adjusted by regulating the position of the tip of the first arm portion 123 in the up-down direction using the fourth ball screw device 125. The position of the tip portion 7 of the cotton swab 6 can be adjusted by making the orientation of the fixed portion 122 oblique relative to the initial position shown by the solid line in FIG. 9 and FIG. 10 and regulating the degree of the tilt.

The control device 90 controls operation of the first and second conveying paths 20 and 30, operation of the first and second moving devices 50 and 60, operation of the chuck device 70, and operation of the position correction device 110.

Now, operation of the examination pretreatment apparatus 40 will be described. As shown in FIG. 1, the control device 90 controls the second conveying belt driving section 35 of the second conveying path 30 to place the second holder 36 in a first position P1. Specifically, the control device 90 controls the second conveying belt driving section 35 to stop conveyance by the second conveying belt 34 when a certain second holder 36 has moved to the first position P1. This places the certain second holder 36 in the first position P1.

The term "certain second holder 36" as used herein refers to a second holder 36 comprising a second container 31 fixed thereto and in which the mucous membrane, an example of a sample, has not been fed yet. The first position P1 is a position within the range over which the examination pretreatment apparatus 40 can move the mucous membrane. According to the present embodiment, the first position P1 is, by way of example, a position where the second holder 36 overlaps the first base portion 51 in the up-down direction Z. When the first holder 26 is moved to a second position P2, the control device 90 drives the fixation mechanism to fix the first container 21 to prevent the first container 21 from rotating.

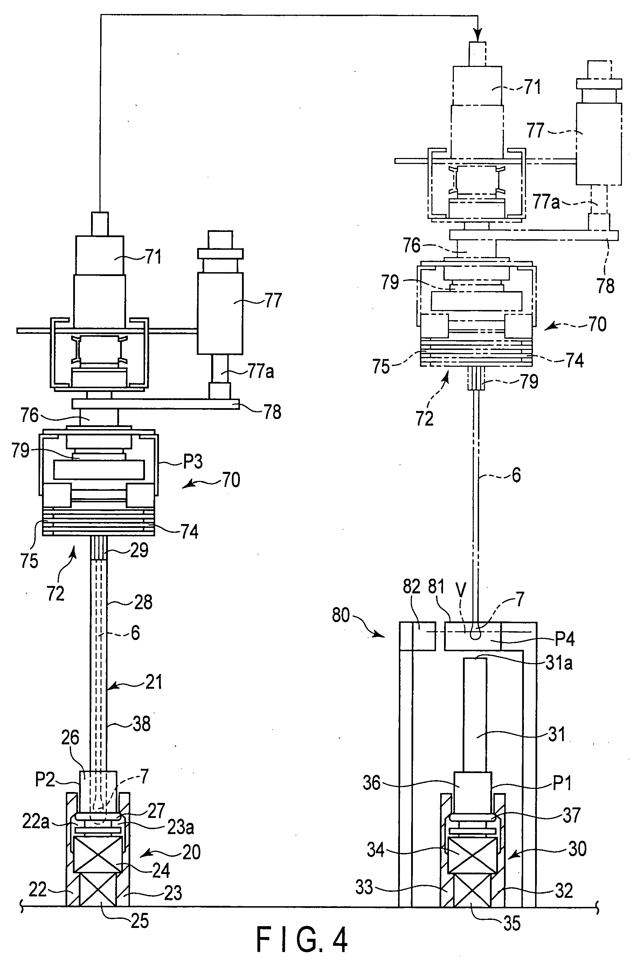

Furthermore, the control device 90 controls the first conveying belt driving section 25 of the first conveying path 20 to place the first holder 26 in the second position P2. In FIG. 4, the position correction device 110 is omitted. Specifically, the control device 90 controls the first conveying belt driving section 25 to stop conveyance by the first conveying belt 24 when a certain first holder 26 has moved to the second position P2. This places the certain second holder 36 in the second position P2.

The term "certain first holder 26" as used herein refers to a first holder 26 with a first container 21 fixed thereto and containing a sample that has not been transferred to the second container 31 yet. The second position P2 is a position where the chuck section 72 can chuck the cap member 29. According to the present embodiment, the second position P2 is, by way of example, a position where the first holder 26 overlaps the first base portion 51 in the up-down direction Z.

FIG. 4 is a schematic diagram showing an operation in which the chuck device 70 transfers the mucous membrane attached to the cotton swab 6 of the cap member 29 to the second container 31. The control device 90 controls the first moving device 50 to move the chuck device 70 to the initial position, while setting the chuck device 70 to an initial state. The initial position is, as shown in FIG. 2, a position where the chuck device 70 is located opposite the cap member 29 of the first container 21 in the second position P2 in the up-down direction Z and where the chuck device 70 is separate from the cap member 29 in the up-down direction Z. Information on the initial position is prestored in the control device 90. The initial state is a state in which the first and second sandwich sections 74 and 75 are open.

Then, the control device 90 controls the second moving device 60 to bring the chuck device 70 down to a lower grip position P3 as shown in FIG. 4. In FIG. 4, the second moving device 60 is omitted. The grip position P3 is a position where the cap member 29 is housed between the first and second housing grooves 74b and 75b in the first and second sandwich sections 74 and 75. Information on the grip position P3 is prestored in the control device 90.

When the chuck device 70 has lowered to the grip position P3, the control device 90 drives the fourth driving motor 77 to allow the cap member 29 to be sandwiched between the first and second sandwich sections 74 and 75. Thus, the cap member 29 is fixed to the chuck section 72. Even after the cap member 29 is fixed to the chuck section 72, the control device 90 further drives the fourth driving motor 77 to rotate the chuck section 72 with the cap member 29 fixed thereto.

Rotation of the chuck section 72 allows the cap member 29 to rotate relative to the container main body 28. The control device 90 stores information on the number of rotations of the cap member 29 needed to remove the cap member 29 from the container main body 28. The control device 90 controls the fourth driving motor 77 and the second moving device 60 to remove the cap member 29 from the container main body 28.

The cap member 29 is threadably engaged with the container main body 28. Thus, rotation of the cap member 29 relative to the container main body 28 allows the cap member 29 to move upward from the container main body 28. Thus, the control device 90 stores information on the amount of upward movement of the cap member 29 relative to the container main body 28 in conjunction with the rotation of the cap member 29 relative to the container main body 28. Based on the information, the control device 90 moves the cap member 29 upward while rotating the cap member 29.

When the cap member 29 has been removed from the container main body 28, the control device 90 moves the chuck section 72 upward to a position where the cotton swab 6 does not interfere with the first container 21 or with the position detection device 80. The position where the cotton swab 6 does not interfere with the first container 21 or with the position detection device 80 is a position where, even when the chuck section 72 is moved in the first or second direction X or Y, the cotton swab 6 does not come into contact with the first container 21 or with the position detection device 80.

When the chuck section 72 has moved to the position where the possible interference is prevented, the control device 90 controls the first and second moving devices 50 and 60 to move the tip portion 7 to a detection position P4.

Now, the position of the cotton swab 6 will be specifically described. The cotton swab 6 is disposed in the center of the cap member 29. Furthermore, the cotton swab 6 extends linearly, and information on the length of the cotton swab 6 is prestored in the control device 90. Thus, information on the position of the tip portion 7 relative to the chuck section 72 is prestored in the control device 90. The position of the tip portion 7 stored in the control device 90 is position information on a state in which the cotton swab 6 extends in a straight line.

The detection position P4 is a position behind an opening 31a in the second container 31 along an insertion direction in which the tip portion 7 of the cotton swab 6 is inserted into the opening 31a. Preferably, the detection position P4 is a position immediately behind the opening 31a along the insertion direction. The first direction position detection section 81 and second direction position detection section 82 of the position detection device 80 are arranged to surround the detection position P4.

That is, when the detection position P4 is set to be a position immediately in front of the opening 31a, the position detection device 80 can detect the position of the tip portion 7 immediately before the tip portion 7 is inserted into the second container 31. According to the present embodiment, the opening 31a faces upward, and the second container 31 extends in the up-down direction Z. Thus, according to the present embodiment, the insertion direction is a direction from an upper side toward a lower side.

That is, the detection position P4 is a position where, as viewed along the insertion direction, the tip portion 7 is positioned inside the opening 31a and where bringing the chuck section 72 down allows the tip portion 7 of the cotton swab 6 to be inserted into the second container 31. The detection position P4 is based on a state in which the cotton swab 6 is not bent.

The cotton swab 6 may be bent. When the cotton swab 6 is bent, an error may be preset between the actual position of the tip portion 7 and the position of the tip portion 7 stored in the control device 90.

The position detection device 80 detects the position, in a plane V perpendicular to the insertion direction, of the tip portion 7 placed in the detection position P4. According to the present embodiment, the insertion direction is the direction from the upper side toward the lower side. Thus, the plane V perpendicular to the insertion direction is a plane defined by the first and the second directions X and Y.

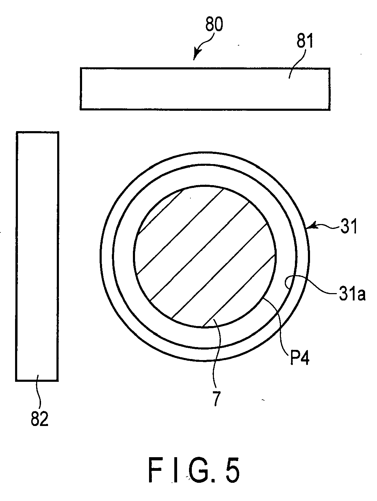

The result of detection, by the position detection device 80, of the position of the tip portion 7 is transmitted to the control device 90. Upon determining that the tip portion 7 appears to be positioned inside the opening 31a as viewed in the insertion direction, the control device 90 drives the second moving device 60 to bring the chuck section 72 down. FIG. 5 is a plan view showing that the tip portion 7 overlaps the inside of the opening 31a in the up-down direction Z.

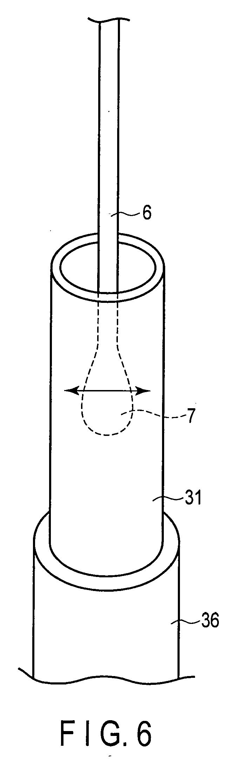

FIG. 6 is a perspective view showing how the tip portion 7 is housed in the second container 31. As shown in FIG. 6, when the tip portion 7 is housed in the second container 31, the control device 90 stops driving the second moving device 60. The control device 90 stores information on a position where the tip portion 7 is immersed in the saline in the second container 31. The information on the position where the tip portion 7 is immersed in the saline in the second container 31 is based on the state in which the cotton swab 6 is not bent.

Then, the control device 90 controls the first moving device 50 to move the tip portion 7 in the first or second direction X or Y by a predetermined length.

When the tip portion 7, immersed in the saline in the second container 31, is moved along the first or second direction X or Y, the mucous membrane attached to the tip portion 7 can be dropped into the second container 31. Thus, the mucous membrane is transferred into the second container 31.

When the mucous membrane attached to the tip portion 7 is dropped into the second container 31, in another example, the position correction device 110 may swing the cotton swab 6 to drop the mucous membrane attached to the tip portion 7.

Subsequently, the control device 90 moves the chuck section 72 upward and then to above a cotton swab recovery box 100. When the chuck section 72 has moved to above the cotton swab recovery box 100, the control device 90 drives the fourth driving motor 77 to move the first and second sandwich sections 74 and 75 to a position where the first and second sandwich sections 74 and 75 are open. When the first and second sandwich sections 74 and 75 move to the position where the first and second sandwich sections 74 and 75 are open, the fixation of the cap member 29 by the chuck section 72 is released. Then, the cap member 29 falls into the cotton swab recovery box 100 under gravity. This allows the cotton swab 6 to be recovered.

The chuck section 72 from which the cotton swab 6 has been removed returns to the initial position and thus to the initial state. The control device 90 drives the fixation mechanism to release the fixation of the first container 21. Then, the first and second containers 21 and 31 positioned next to the first and second containers 21 and 31 used for the above-described treatment are placed in the first and second positions P1 and P2, with the above-described operations repeated. Thus, the mucous membranes contained in the first containers 21 are transferred into the second containers 31 in order.

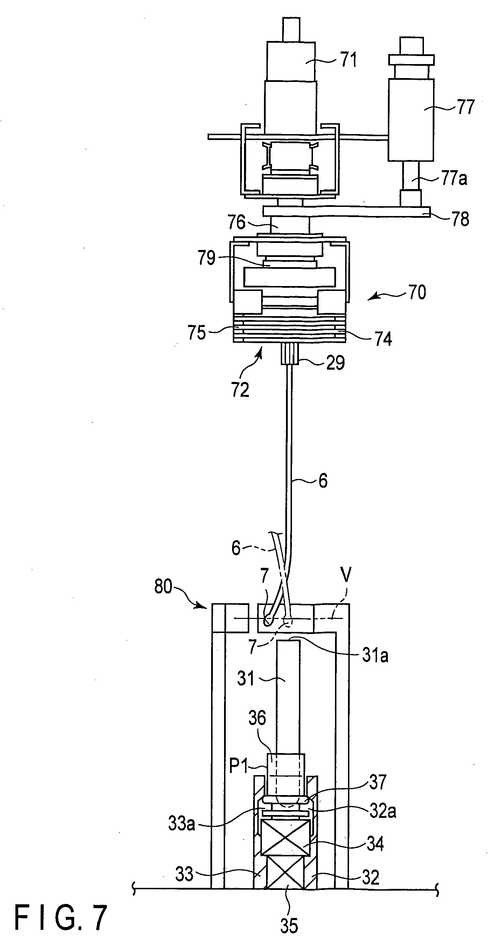

Now, an operation performed by the examination pretreatment apparatus 40 when the cotton swab 6 is bent will be described. FIG. 7 is a schematic diagram showing an operation performed by the examination pretreatment apparatus 40 when the cotton swab 6 is bent. FIG. 7 shows that the tip portion 7 of the cotton swab 6 has moved to the detection position P4. An operation performed by the position detection device 80 to detect the tip portion 7 of the cotton swab 6 is the same as the operation described above with reference to FIG. 4.

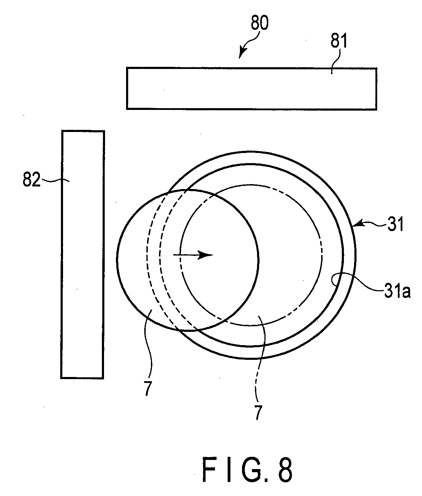

FIG. 8 is a plan view similar to FIG. 5, showing that the tip portion 7 of the bent cotton swab 6 has moved to the vicinity of the detection position P4. Now, the length, along the up-down direction Z, of the first and second direction position detections 81 and 82 will be described. First, as described above, the control device 90 stops driving the second moving device 60 when the tip portion 7 of the unbent cotton swab 6 reaches the vicinity of the detection position P4. Thus, when the cotton swab 6 is bent as in this description, the tip portion 7 of the cotton swab 6 may be positioned above the detection position P4 when the second moving device 60 is stopped.

However, since the cotton swab 6 is housed in the container main body 28 of the first container 21, the degree of bending of the cotton swab 6 falls within the range in which the bent cotton swab 6 can be housed in the container main body 28. Thus, the range over which the tip portion 7 of the cotton swab 6 may be positioned can be predetermined.

According to the present embodiment, by way of example, the detection position P4, that is, the position of the tip portion 7 of the unbent cotton swab 6, is set to be the center, in the up-down direction Z, of the first direction position detection section 81 and the second direction position detection section 82. The length, along the up-down direction Z, of the first and second direction position detection sections 81 and 82 is appropriate to allow detection of the range over which the tip portion 7 of the bent cotton swab 6 may be positioned as predicted as described above.

In this case, the tip portion 7 has moved to the vicinity of the detection position P4. This is because bending of the cotton swab 6 precludes the tip portion 7 from reaching the detection position P4 when the second moving device 60 is stopped under the control of the control device 90.

The operation of the examination pretreatment apparatus 40 will be described again. In this description, the cotton swab 6 is bent, and thus, the tip portion 7 partly overlaps the outside of the opening 31a of the second container 31 in the insertion direction.

In this state, when the chuck section 72 lowers, the tip portion 7 fails to be housed inside the second container 31. Thus, the control device 90 controls the position correction device 110, specifically the fourth ball screw device 125, to move the position of the chuck section 72 so that the tip portion 7 is positioned inside the opening 31a of the second container 31 as viewed in the insertion direction.

Specifically, the control device 90 controls the fourth ball screw device 125 so that the tip portion 7 is positioned inside the opening 31a in the second container 31, based on the position of the tip portion 7 in the plane V detected by the position detection device 80.

In FIG. 8, an alternate long and two short dashes line shows a state in which the position of the chuck section 72 has been moved such that the tip portion 7 is positioned inside the opening 31a in the second container 31 as viewed in the insertion direction. Similarly, in FIG. 7, an alternate long and two short dashes line shows a state in which the position of the chuck section 72 has been moved such that the tip portion 7 is positioned inside the opening 31a in the second container 31 as viewed in the insertion direction.

When the tip portion 7 has moved to the inside of the opening 31a as viewed in the insertion direction, as a result of the control of the fourth ball screw device 125, the control device 90 controls the second moving device 60 to bring the chuck section 72 down to place the tip portion 7 inside the second container 31. The subsequent operation is the same as the corresponding operation described with reference to FIG. 5.

When inserted into the second container 31, even the bent cotton swab 6 is extended upon coming into the inner surface of the second container 31. The position where the tip portion 7 is immersed in the saline in the second container 31 is a position set so that the tip portion can be immersed in the saline even when the bent cotton swab is extended as described above.

The length from the chuck section 72 to the tip portion 7 measured when the cotton swab 6 extends straight instead of being bent is larger than the length from the chuck section 72 to the tip portion 7 measured when the cotton swab 6 is bent. Thus, the position where the tip portion 7 is immersed in the saline which position is stored in the control device 90 is determined so that the tip portion 7 of the bent cotton swab 6 can be immersed in the saline when the bent cotton swab 6 is extended upon coming into contact with the inner surface of the second container 31. This allows the tip portion 7 of the unbent, straight cotton swab 6 to be also immersed in the saline.

Thus, the examination pretreatment apparatus 40 detects the position of the tip portion 7 of the cotton swab 6 immediately before the tip portion 7 is placed in the second container 31 and corrects the position of the cotton swab 6 so that the tip portion 7 is located inside the opening 31a in the second container 31. Specifically, the correction is made so that the tip portion 7 is positioned inside the opening 31a as viewed in the insertion direction. Thus, even when the cotton swab 6 is bent, the tip portion 7 of the cotton swab 6 is automatically placed in the second container 31. This allows the mucous membrane to be efficiently transferred from the first container 21 to the second container 31.

According to the present embodiment, the cotton swab is an example of a rod material according to the present invention. The chuck device 70 and the first and second moving devices 50 and 60 form an example of moving means according to the present invention. The position detection device 80 is an example of position detection means according to the present invention. The control device 90 is an example of position detection means according to the present invention.

The state where the tip portion is positioned inside the opening as viewed in the insertion direction indicates that the whole tip portion 7 is located inside the opening and that, even when the tip portion 7 is moved in the insertion direction, hindrance of the insertion is inhibited which may result from, for example, contact between the tip portion and an edge of the opening.

Furthermore, according to the present embodiment, the first container 21 comprises the container main body 28 and the cap member 29. The cap member 29 is engaged with the container main body 28 in a threaded manner. Thus, in order to be removed from the container main body 28, the cap member 29 conventionally needs to be rotated relative to the cap member 29.

In another example of the first container, the cap member may be structured to be integrally fixed to the container main body by insertion instead of being engaged with the container main body in a threaded manner. In this case, the chuck device need not have a function to rotate the cap member.

## Claims

1. An examination pretreatment apparatus comprising:
moving means (70,50,60) for placing a tip portion (7) of a rod member (6) housed in a first container (21) in a second container (31) different from the first container (21) through an opening (31 a) in the second container (31);
position detection means (80) for detecting a position of the tip portion (7);
position correction means (110) for correcting the position of the tip portion (7) by tilting the moving means (60); and
control means (90) configured to control the position correction means (110) so that the tip portion (7) is moved into the second container (31), based on a result of detection by the position detection means (80), and control the position correction means (110) so as to swing the tip portion (7) inside the second container (31) after the tip portion (7) is housed in the second container (31).

2. The examination pretreatment apparatus according to claim 1, **characterized in that** the position detection means (80) detects the position of the tip portion (7) in a plane perpendicular to an insertion direction in which the tip portion (7) is inserted into the opening (31 a) in the second container (31) at a detection position set in front of the opening (31 a) along the insertion direction, and
the control means (90) controls the position correction means (110) to make the position of the tip portion (7) in the plane corrected so that the tip portion (7) is positioned inside the opening (31 a) in the second container (31) as viewed in the insertion direction, based on the result of the detection by the detection means (80), and after the position is corrected, the control means (90) controls the moving means (70,50,60) so that the tip portion (7) is moved in the insertion direction.

3. The examination pretreatment apparatus according to claim 1 or 2, **characterized in that**
the control means (90) is for controlling the moving means (70, 50, 60) so as to move the tip portion (7) inside the second container (31).

## Patentansprüche

1. Untersuchungs-Vorbereitungsvorrichtung umfassend:
Bewegungsmittel (70, 50, 60) zum Platzieren eines in einem ersten Behälter (21) aufgenommenen Spitzenabschnitts (7) eines Stäbchenteils (6) in einen zu dem ersten Behälter (21) verschiedenen zweiten Behälter (31) durch eine Öffnung (31 a) in dem zweiten Behälter (31);
ein Positionserkennungsmittel (80) zum Erkennen einer Position des Spitzenabschnitts (7);
ein Positionskorrekturmittel (110) zum Korrigieren der Position des Spitzenabschnitts (7) durch Neigen der Bewegungsmittel (60); und
ein Steuerungsmittel (90), das eingerichtet ist
zum Steuern des Positionskorrekturmittels (110), sodass der Spitzenabschnitt (7), basierend auf dem Ergebnis einer Erkennung durch das Positionserkennungsmittel (80), in den zweiten Behälter (31) verschoben wird, und
zum Steuern des Positionskorrekturmittels (110), um den Spitzenabschnitt (7) innerhalb des zweiten Behälters (31) zu verschwenken, nachdem der Spitzenabschnitt (7) in dem zweiten Behälter (31) aufgenommen ist.

2. Untersuchungs-Vorbereitungsvorrichtung nach Anspruch 1, **dadurch gekennzeichnet, dass** das Positionserkennungsmittel (80) die Position des Spitzenabschnitts (7) in einer Ebene, die senkrecht zu einer Einsetzrichtung liegt, in der der Spitzenabschnitt (7) in die Öffnung (31a) in den zweiten Behälter (31) eingesetzt wird, an einer bezüglich der Einsetzrichtung vor der Öffnung (31a) liegenden Erkennungsposition erkennt, und
das Steuerungsmittel (90) das Positionskorrekturmittel (110) basierend auf dem Ergebnis einer Erkennung durch das Erkennungsmittel (80) steuert, um die Position des Spitzenabschnitts (7) in der Ebene zu korrigieren, sodass der Spitzenabschnitt (7) in Einsetzrichtung betrachtet innerhalb der Öffnung (31 a) in dem zweiten Behälter (31) positioniert wird, und das Steuerungsmittel (90) die Bewegungsmittel (70, 50, 60) steuert, sodass der Spitzenabschnitt (7) in die Einsetzrichtung verschoben wird, nachdem die Position korrigiert wurde.

3. Prüfvorbehandlungsvorrichtung nach einem der Ansprüche 1 oder 2, **dadurch gekennzeichnet, dass** das Steuerungsmittel (90) zum Steuern der Bewegungsmittel (70, 50, 60) ausgebildet sind, um den Spitzenabschnitt (7) in den zweiten Behälter (31) zu verschieben.

## Revendications

1. Appareil de prétraitement d'examen comprenant :
des moyens de déplacement (70, 50, 60) pour placer une partie de pointe (7) d'un élément en forme de tige (6) logé dans un premier récipient (21) dans un deuxième récipient (31) différent du premier récipient (21) à travers une ouverture (31 a) dans le deuxième récipient (31) ;
un moyen de détection de position (80) pour détecter une position de la partie de pointe (7) ;
un moyen de correction de position (110) pour corriger la position de la partie de pointe (7) en inclinant le moyen de déplacement (60) ; et
un moyen de commande (90) configuré pour
commander le moyen de correction de position (110) de sorte que la partie de pointe (7) soit déplacée dans le deuxième récipient (31), en fonction d'un résultat de détection provenant du moyen de détection de position (80), et
commander le moyen de correction de position (110) de manière à faire osciller la partie de pointe (7) à l'intérieur du deuxième récipient (31) après que la partie de pointe (7) est logée dans le deuxième récipient (31).

2. Appareil de prétraitement d'examen selon la revendication 1, **caractérisé en ce que** le moyen de détection de position (80) détecte la position de la partie de pointe (7) dans un plan perpendiculaire à une direction d'insertion dans laquelle la partie de pointe (7) est insérée dans l'ouverture (31 a) dans le deuxième récipient (31) à une position de détection placée devant l'ouverture (31 a) le long de la direction d'insertion, et
le moyen de commande (90) commande le moyen de correction de position (110) pour entraîner la correction de la position de la partie de pointe (7) dans le plan de sorte que la partie de pointe (7) soit positionnée à l'intérieur de l'ouverture (31 a) dans le deuxième récipient ( 31) telle que vue dans la direction d'insertion, en fonction du résultat de la détection provenant du moyen de détection (80), et après que la position est corrigée, le moyen de commande (90) commande les moyens de déplacement (70, 50, 60) de sorte que la partie de pointe (7) soit déplacée dans la direction d'insertion.

3. Appareil de prétraitement d'examen selon la revendication 1 ou 2, **caractérisé en ce que** le moyen de commande (90) sert à commander les moyens de déplacement (70, 50, 60) de manière à déplacer la partie de pointe (7) à l'intérieur du deuxième récipient (31).
